# EUROPEAN PATENT APPLICATION

(11) **EP 2 671 568 A1**
(43) Date of publication of application: **11.12.2013**
(21) Application number: 12741948.9
(22) Date of filing: 30.01.2012
(51) Int. Cl.: A61K 9/107, A61K 47/06, A61K 47/10, A61K 47/14, A61K 47/34, B01J 13/00

(54) **DERMAL COMPOSITION COMPRISING POLYMERIC REVERSED MICELLE, AND METHOD FOR PRODUCING SAME**

(30) Priority: 31.01.2011 JP 2011018612
(71) Applicant: MARUHO Co., Ltd., Osaka-shi, Osaka 531-0071 (JP)
(72) Inventor: AKAMATSU, Ryo, Kyoto-shi Kyoto 600-8815 (JP); SAKAGUCHI, Tomoki, Kyoto-shi Kyoto 600-8815 (JP); SAKIYAMA, Hiroki, Kyoto-shi Kyoto 600-8815 (JP)
(74) Representative: Pfenning, Meinig & Partner GbR
(86) International application number: PCT/JP2012/051965
(87) International publication number: WO 2012/105485

(57) **Abstract**

The present invention provides a dermal composition comprising a polymeric reversed micelle that allows a water-soluble drug to be efficiently encapsulated and that is superior in percutaneous absorptiveness and very safe, and provides a method that can produce the composition in simple steps. The dermal composition comprises a polymeric reversed micelle composed of an amphipathic polymer having a hydrophilic segment and a hydrophobic segment, wherein the polymeric reversed micelle has a configuration in which the hydrophilic segment is the core and the hydrophobic segment is the shell, and a water-soluble drug is encapsulated therein. The composition can be produced by blending an oil phase comprising the amphipathic polymer in an oily base agent with an aqueous phase comprising the water-soluble drug in an aqueous solvent, or by blending an oily base agent with an aqueous phase comprising the amphipathic polymer and the water-soluble drug in an aqueous solvent.

## Description

### TECHNICAL FIELD

The present invention relates to an external preparation containing a micelle composed of a polymer. More specifically, the present invention relates to a dermal composition containing a polymeric reversed micelle having a hydrophobic shell and a hydrophilic core, and a method for producing the composition.

### BACKGROUND ART

Polymeric micelle is created as a drug delivering system (DDS) of an anticancer agent. At present, there exist plural developed products (parenteral injections) at a clinical test stage. In recent years, fields to which polymeric micelle is to be applied have been spreading. For example, polymeric micelle has been used to improve drugs in intestinal absorptiveness.
A polymeric micelle having a hydrophilic shell and a hydrophobic core has been actively researched. Thus, many known techniques thereabout are present. In a part of the techniques, the application thereof to percutaneous absorption has been tried.

However, it cannot be said that the hydrophilic shell is high in skin-permeability. The hydrophobic core has a problem of being unsuitable for a matter that a water-soluble drug is enclosed and held in the core. Any water-soluble drug generally shows a slight percutaneous absorptiveness because of a high polarity thereof. Thus, it is desired to develop a technique for promoting the percutaneous absorptiveness thereof. However, the polymeric micelle having a hydrophilic shell and a hydrophobic core has a limit to skin permeability and drug encapsulating-in performance.

Some polymeric reversed micelles each having a hydrophobic shell and a hydrophilic core have been developed. However, under the present circumstances, the micelles are not applied to percutaneous absorption.

For example, Patent Document 1 discloses fine particles each having, at the inside thereof, a hydrophilic moiety in which a protein can be efficiently encapsulated, and a method for producing the dispersion of the fine particles. However, an object of this technique is to produce fine particles for being orally administered or enterally administered, or being administered by injection. Patent Document 1 does not disclose the production of any polymeric reversed micelle suitable for percutaneous absorption.

The producing method according to Patent Document 1 includes not only the step (a) of blending an aqueous solvent containing a hydrophilic active substance with a water-immiscible organic solvent in which an amphipathic polymer is dissolved, thereby forming a reversed-phase emulsion but also the step (b) of introducing the reversed-phase emulsion into a liquid phase containing a surface modifier, and the step (c) of removing the water-immiscible organic solvent. Therefore, this method requires much labor, and moreover, a solvent used as the water-immiscible organic solvent is a solvent included in class 2 or 3 according to the Residual Solvent Guideline, for example, ethyl acetate or chloroform. Thus, there is caused a problem that the method is unfavorable for safety even when the removal step is performed after the use of the solvent.

### PRIOR ART DOCUMENT

### PATENT DOCUMENT

Patent Document 1: JP-A-2007-326833

### SUMMARY OF THE INVENTION

### PROBLEMS TO BE SOLVED BY THE INVENTION

Accordingly, an object of the present invention is to provide a dermal composition which allows a water-soluble drug to be efficiently encapsulated therein, and is excellent in percutaneous absorptiveness and high in safety; and a method for producing this dermal composition through a simple step.

### MEANS FOR SOLVING THE PROBLEMS

In order to solve the problems, the present inventors have made eager investigations to succeed in obtaining a composition containing a polymeric reversed micelle in which a water-soluble drug is encapsulated by a simple operation of blending an oil phase comprising an amphipathic polymer in an oily base agent with an aqueous phase comprising the water-soluble drug in an aqueous solvent, or blending an oily base agent with an aqueous phase comprising an amphipathic polymer and the water-soluble drug in an aqueous solvent. The present inventors have further verified that when this composition is applied to the skin, the water-soluble drug exhibits a high percutaneous absorptiveness. Thus, the problems have been solved.

Accordingly, the present invention is a method for producing a dermal composition comprising a polymeric reversed micelle in which a water-soluble drug is encapsulated, comprising:
the step of blending an oil phase comprising an amphipathic polymer, which is having a hydrophilic segment and a hydrophobic segment, in an oily base agent; with an aqueous phase comprising a water-soluble drug in an aqueous solvent, or
blending an oily base agent with an aqueous phase comprising the above-defined amphipathic polymer and a water-soluble drug in an aqueous solvent,
thereby producing the polymeric reversed micelle in which the hydrophilic segment is a core, the hydrophobic segment is a shell and the water-soluble drug is encapsulated.

The present invention is also a dermal composition that can be produced by the method. The dermal composition comprises a polymeric reversed micelle comprising an amphipathic polymer having a hydrophilic segment and a hydrophobic segment, wherein the polymeric reversed micelle is a micelle in which the hydrophilic segment is a core, the hydrophobic segment is a shell and a water-soluble drug is encapsulated.

### EFFECT OF THE INVENTION

In the polymeric reversed micelle in the dermal composition according to the present invention, its hydrophilic segment is a core and its hydrophobic segment is a shell. Thus, a water-soluble drug can stably be encapsulated in the hydrophilic core, and further the water-soluble drug can be improved in percutaneous absorptiveness by the hydrophobic shell, which has a high affinity with the skin, which has, as its outermost layer, a hydrophobic horny layer. In conclusion, a slight percutaneous absorptiveness of the water-soluble drug, which is caused by a high polarity of the drug, can be masked so that the water-soluble drug can be efficiently delivered to the inside of the skin.
As the oily base agent, a base agent usable widely for dermal pharmaceutical preparations can be used; thus, the dermal composition of the present invention is high in safety.
The method according to the present invention is attainable merely by blending an oily base agent comprising an amphipathic polymer with an aqueous solution comprising a water-soluble drug, or blending an oily base agent with an aqueous solution comprising an amphipathic polymer and a water-soluble drug. For this reason, the process thereof is very simple and easy.

### MODE FOR CARRYING OUT THE INVENTION

In the present invention, the amphipathic polymer is a polymer containing a hydrophilic segment and a hydrophobic segment and having a property that in an oil the hydrophilic segment constitutes a core (inner layer) and the hydrophobic segment constitutes a shell (outer layer) so that the core and the shell form a polymeric reversed micelle. The polymer further has a property that a water-soluble drug, or an aqueous solution containing a water-soluble drug is encapsulated in the inner layer. Examples of the amphipathic polymer according to the present invention include not only any amphipathic block copolymer in which a hydrophilic polymer is linked with a hydrophobic polymer (such as any polyoxyethylene polyoxypropylene glycol, or any polyoxyethylene polyoxypropylene alkyl ether) but also any amphipathic graft copolymer in which a monomer or polymer having one out of hydrophilicity and hydrophobicity is linked, as a branched component, in a comb-tooth form with a polymer having the other, and any amphipathic polymer in which only a hydrophilic group or a lipophilic group is a polymer.
The hydrophobic segment is not particularly limited. Examples thereof include aliphatic acid polyesters, polyamino acids, polyoxyalkylene glycols (from which polyoxymethylene glycol and polyoxyethylene glycol are excluded), such as polyoxypropylene glycol and polyoxybutylene glycol, polystyrene, polyvinyl ether, hydrocarbons, higher alcohols, and aliphatic acids.
The hydrophilic segment is not particularly limited. Examples thereof include polyoxymethylene glycol, polyoxyethylene glycol, polyvinyl pyrrolidone, polyvinyl alcohol, polyethyleneimine, polyacrylic acid, polymethacrylic acid, polyamino acids, polyoxyethylene glycerin, sulfonic acids (sulfonates), phosphoric acid (phosphates), carboxylic acids (carboxylates), amines, and quaternary ammonium (quaternary ammonium salts).

Preferred examples of the amphipathic polymer in the present invention include:
polyoxyethylene polyoxypropylene glycols, and polyoxyethylene polyoxypropylene alkyl ethers, which are amphipathic block copolymers;
aliphatic acid esters of any polyoxyethylene glyceryl ether, and polyoxyethylene hydrogenated castor oils, in each of which only a hydrophilic group is a polymer; and
polyoxypropylene quaternary ammonium salts, in each of which only a lipophilic group is a polymer.
Particularly preferred examples of the amphipathic polymer include polyoxyethylene polyoxypropylene glycols, polyoxyethylene polyoxypropylene alkyl ethers, and polyoxypropylene quaternary ammonium salts.

Preferred examples of the polyoxyethylene polyoxypropylene glycols are polyoxyethylene polyoxypropylene glycols each selected from the group consisting of polyoxyethylene (8) polyoxypropylene (55) glycol, polyoxyethylene (30) polyoxypropylene (35) glycol, polyoxyethylene (24) polyoxypropylene (25) glycol, polyoxyethylene (12) polyoxypropylene (35) glycol, polyoxyethylene (10) polyoxypropylene (65) glycol, polyoxyethylene (3) polyoxypropylene (17) glycol, and polyoxyethylene (5) polyoxypropylene (30) glycol. Particularly preferred examples thereof include polyoxyethylene (8) polyoxypropylene (55) glycol, polyoxyethylene (10) polyoxypropylene (65) glycol, polyoxyethylene (24) polyoxypropylene (25) glycol, and polyoxyethylene (12) polyoxypropylene (35) glycol.
Preferred examples of the polyoxyethylene polyoxypropylene alkyl ethers include polyoxyethylene (17) polyoxypropylene (17) butyl ether, polyoxyethylene (30) polyoxypropylene (30) butyl ether, polyoxyethylene (37) polyoxypropylene (38) butyl ether, polyoxyethylene (25) polyoxypropylene (25) lauryl ether, polyoxyethylene (3) polyoxypropylene (34) stearyl ether, and polyoxyethylene (10) polyoxypropylene (20) decyl tetradecyl ether. Particularly preferred examples thereof include polyoxyethylene (10) polyoxypropylene (20) decyl tetradecyl ether.

In the present invention, preferred examples of the amphipathic polymer in which only a hydrophilic group (hydrophilic segment) is a polymer include aliphatic acid esters of any polyoxyethylene glyceryl ether. Preferred is polyoxyethylene glyceryl isostearate, and particularly preferred are polyoxyethylene (10) glyceryl diisostearate, and polyoxyethylene (20) glyceryl triisostearate.
Other preferred examples of the amphipathic polymer, in which only a hydrophilic group is a polymer, include polyoxyethylene hydrogenated castor oils. Particularly preferred is polyoxyethylene hydrogenated castor oil 10.

In the present invention, preferred examples of the amphipathic polymer in which only a lipophilic group (hydrophobic segment) is a polymer include polyoxypropylene quaternary ammonium salts. Particularly preferred is polyoxypropylene (25) diethylmonium chloride.

About the amphipathic polymer, only one species thereof may be used alone, or plural species thereof may be used together. The amphipathic polymer may be a nonionic polymer, or an ionic polymer, such as a polyoxypropylene quaternary ammonium salt. A low-molecular-weight amphipathic substance may be together used.

The molecular weight of the amphipathic polymer in the present invention is not particularly limited, and is preferably from 500 to 10,000 (examples of the polymer corresponding thereto include the amphipathic polymers described specifically in paragraphs [0014] to [0017]), more preferably from 600 to 8, 000, in particular preferably from 800 to 5,000, even more preferably from 1,000 to 4,500. This molecular weight denotes weight-average molecular weight.

It is preferred to add the amphipathic polymer to the dermal composition in a content of 0.001 to 90% by weight of the composition. The content of the amphipathic polymer is more preferably from 0.1 to 80% by weight, in particular preferably from 5 to 60% by weight, even more preferably from 15 to 50% by weight, though these values may be varied in accordance with the kind of the amphipathic polymer and the critical micelle concentration (CMC) thereof.

The composition of the present invention contains a reversed micelle formed by the amphipathic polymer (polymeric amphipathic molecules). Accordingly, the micelles are not easily broken even when the micelles are diluted in an administration route thereof. Thus, the composition is very good as a dermal external preparation. In other words, a low-molecular-weight micelle formed by use of low-molecular-weight amphipathic molecules (surfactant) has a high critical micelle concentration; thus, when the micelles are applied to the skin, the micelles are diluted in its horny layer so that many micellar particles are broken. However, the high-molecular-weight amphipathic polymer has a low critical micelle concentration. Accordingly, even when the micelles are diluted in the horny layer, the micellar particles are not easily broken. For this reason, the polymeric reversed micelle related to the present invention is very suitable for a carrier which permeates the horny layer in the state that a drug is encapsulated therein.

In order to make better the skin permeability of the polymeric reversed micelle, it is preferred to make the polymeric reversed micelle to have a particle diameter smaller than gaps (50 to 70 nm) between horny cells. The producing method according to the present invention also makes it possible to produce a polymeric reversed micelle having a particle diameter less than 50 nm, which is advantageous for percutaneous absorption, by a simple operation of combining an oil phase (or oily base agent) with an aqueous phase, and stirring the combination. Furthermore, conventional fine particle species, a typical example thereof being liposome, is low in drug encapsulating-in percentage when in the form of particles having a particle diameter less than 50 nm. However, the polymeric reversed micelle related to the present invention is high in drug encapsulating-in percentage when the micelle also has a particle diameter less than 50 nm. From this viewpoint also, therefore, the composition containing the polymeric reversed micelle related to the present invention is very suitable for a dermal external preparation.
The particle diameter of the polymeric reversed micelle related to the present invention is measurable by particle diameter analysis based on dynamic light scattering.

The oily base agent related to the present invention needs to be an oily base agent immiscible with water or slightly miscible with water (the polarity value α of the organic compound that is based on the Organic Conceptual Diagram: about 0 to 60°), and be further an oily base agent in which the used amphipathic polymer easily forms a reversed micelle.

The oily base agent is not particularly limited, and is preferably an oily base agent that has widely been used for dermal pharmaceutical preparations, and has an established safety. Examples thereof include hydrocarbons such as liquid paraffin, and squalane; aliphatic acid esters such as isopropyl myristate, diethyl sebacate, and diisopropyl adipate; triglycerides such as glycerin triisooctanate, and triglyceride of any middle-chain aliphatic acid; higher alcohols such as octyldodecanol, hexyldecanol, and isostearyl alcohol; aliphatic acids such as isostearic acid; and other highly polar oils such as ethylene glycol salicylate, propylene carbonate, crotamiton and triacetin.
The oily base agent is more preferably any one selected from hydrocarbons, aliphatic acid esters, higher alcohols, and highly polar oils. The oily base agent is preferably a liquid oily base agent. Particularly preferred examples of the liquid oily base agent include liquid paraffin, isopropyl myristate, diethyl sebacate, diisopropyl adipate, octyldodecanol, triacetin, and ethylene glycol salicylate.
About the oily base agent, only one species thereof may be used alone, or two or more thereof may be used together.

It is preferred to add the oily base agent to the dermal composition in a content of 5 to 95% by weight of the composition. The content of the oily base agent is more preferably from 10 to 90% by weight, in particular preferably from 20 to 85% by weight, even more preferably from 35 to 80% by weight.

The oil phase related to the present invention is a phase in which the above-mentioned amphipathic polymer is contained in the oily base agent. The oil phase may be prepared by adding the amphipathic polymer to the oily base agent, and blending these with each other. In the oil phase, the amphipathic polymer forms a polymeric reversed micelle in which its hydrophilic segment is centrally positioned and its hydrophobic segment is exteriorly positioned.
By adding, to this oil phase, an aqueous phase containing a water-soluble drug and blending these with each other, the water-soluble drug (or an aqueous solution of the water-soluble drug) can be encapsulated in the polymeric reversed micelle.

The aqueous phase related to the present invention is a phase in which a water-soluble drug (or a water-soluble drug and the amphipathic polymer) is dissolved in an aqueous solvent (water, a water-soluble base agent, or water and a water-soluble base agent). The water-soluble base agent is preferably, for example, a polyhydric alcohol such as glycerin, 1,3-butylene glycol or propylene glycol, or polyethylene glycol.

It is preferred to add the aqueous solvent to the dermal composition in a content of 0.1 to 50% by weight of the composition. The content of the aqueous solvent is more preferably from 1 to 40% by weight, in particular preferably from 2 to 30% by weight, even more preferably from 3 to 20% by weight.

The water-soluble drug related to the present invention is not particularly limited as far as the drug is a drug having water-solubility. Preferred examples of the water-soluble drug include tranexamic acid, vitamins, amino acids, (poly)saccharides, polysulfated chondroitin sulfate, polysulfated mucopolysaccharides, and sodium hyaluronate. The reversed micelle related to the present invention can encapsulate therein the water-soluble drug whether the drug is large or small in molecular weight. For example, the micelle can encapsulate therein a water-soluble drug having a molecular weight of 100,000 or less, 10,000 or less, or 1,000 or less.

It is preferred to add the water-soluble drug to the dermal composition in a content of 0.0001 to 20% by weight of the composition. The content of the water-soluble drug is more preferably from 0.01 to 5% by weight, in particular preferably from 0.05 to 3% by weight.

In the producing method of the present invention, the blend ratio between the individual components should be appropriately adjusted not to cause the precipitation of the drug, an increase in the particle diameter of the reversed micelle nor the advance of ununiformity in the pharmaceutical preparation in accordance with the species of the used water-soluble drug, amphipathic polymer and oily base agent.
An example of a preferred formulation of the dermal composition according to the present invention is a dermal composition in which the content of the amphipathic polymer is 15 to 50% by weight, the content of the oily base agent is 35 to 80% by weight, the content of the aqueous solvent is 3 to 20% by weight and the content of the water-soluble drug is 0.05 to 3% by weight.

An example of the producing process according to the present invention is a method of blending an oil phase prepared by adding an amphipathic polymer to an oily base agent and blending these with each other, with an aqueous phase prepared by adding a water-soluble drug to an aqueous solvent and dissolving the drug in the solvent.
Another example of the producing process according to the present invention is a method of blending an oily base agent with an aqueous phase prepared by adding a water-soluble drug and an amphipathic polymer to an aqueous solvent and blending these with each other.
As to whether the amphipathic polymer is added to the oily base agent or to the aqueous solvent, which one permits the amphipathic polymer to be dissolvable or meltable/miscible should be selected. At the time of, for example, the preparation of the oil phase, the system may be heated if necessary.
The blending may be attained, using a stirring device such as a stirrer. The stirring speed and the stirring period are not particularly limited. For example, the stirring may be performed at a stirring speed of 50 to 2000 rpm for a stirring period of about 1 minute to 12 hours. If necessary, a homogenizer, a micro fluidizer or some other apparatus may be used.

In the producing method according to the present invention, a composition obtained by blending the oil phase (or the oily base agent) with the aqueous phase is in a form from liquid forms to semi-solid forms. Accordingly, in the case of desiring to obtain a liquid to a semi-solid dermal composition, a composition in a desired dosage-form can be directly produced. When a semi-solid or solid dosage-form is desired, the resultant composition should be further worked into a desired dermal external preparation.
For example, in a case where just after the composition is produced the dosage-form thereof is a reversed-micelle-containing dispersed liquid form but a dosage-form from semi-solid forms to solid forms is desired, the following step should be performed: for example, a step of dispersing the resultant dispersed liquid into a base agent such as white vaseline; or a step of emulsifying the dispersed liquid in the form of droplets into water or the like. When a gelatinous dosage-form is desired, for example, a step of gelatinizing the resultant dispersed liquid with a tackifier should be performed. These components may be beforehand added, at the time of the preparation of the polymeric reversed micelle, to the preparing system.

As described above, the dermal composition according to the present invention can be directly produced by the producing method described hereinbefore, or produced by post-working a composition obtained by the producing method. Thus, the oily base agent or aqueous solvent used in the producing method may be contained in the dermal composition according to the present invention.
If necessary, the dermal composition according to the present invention may contain components to be blended into any ordinary dermal composition, such as a colorant, a perfume, a pigment, a pH adjustor, a preservative, an antioxidant, and an ultraviolet absorbent. These may be added after a composition containing a polymeric reversed micelle is produced by the producing method according to the present invention. Alternatively, an oil phase (or oily base agent) or an aqueous phase to which these components are beforehand added may be prepared; or when an oil phase (or oily base agent) is combined with an aqueous phase, these components may be added to the combining system.
The dermal composition of the present invention is usable as a cosmetic product or a sanitary product, other than an external preparation for medicine.

### EXAMPLES

Hereinafter, examples of the present invention will be described. However, the present invention is not limited by these examples.

### [Example 1]

In this Example, an amphipathic polymer and an oily base agent which had been actually used for dermal compositions and had been established in safety were used.
Compositions were each produced by stirring an oil phase prepared by adding the amphipathic polymer into the oily base agent and then blending these with each other; and an aqueous phase prepared by adding a water-soluble drug to an aqueous solvent and dissolving the drug in the solvent.
Furthermore, compositions were each produced by stirring an oily base agent and an aqueous phase prepared by adding a water-soluble drug and an amphipathic polymer into an aqueous solvent and blending these with each other.
After the production of each of the compositions, it was verified by the following methods that a reversed micelle was produced in which its hydrophilic segment was a core and its hydrophobic segment was a shell: a diluting method (water droplets were dropped to the composition, and then it was verified from a difficulty of the dispersion of the droplets that the outside phase was the oily base agent) and particle diameter analysis according to the dynamic light scattering method. If the water-soluble drug leaked to the outside phase (oil phase), the drug would have precipitated. Thus, it was verified with the naked eye and through microscopic observation that no precipitation was observed, so that it was verified that the water-soluble drug was encapsulated in the core of the reversed micelle.

Furthermore, investigations were made about oily base agent species and amphipathic polymer species, and combinations thereof particularly suitable for obtaining polymeric reversed micelles each having a particle diameter smaller (less than 50 nm) than gaps (50 to 70 nm) between horny cells. Furthermore, the respective volumes of the oily base agent species, the amphipathic polymer species, and an aqueous solvent were adjusted to yield compositions each containing a polymeric reversed micelle having a particle diameter less than 50 nm.
In Table 1 are together shown components particularly suitable for each desired composition, and a blend ratio therebetween. The water-soluble drug used to be encapsulated in each of the polymeric reversed micelles was tranexamic acid. About any block copolymer in Table 1, polyoxyethylene is abbreviated to POE; and polyoxypropylene to POP.
The production of each of the compositions shown in Table 1 was attained by adding an aqueous solution of tranexamic acid (aqueous phase) to an oil phase in which one or two of the amphipathic polymers were dissolved in one of the oily base agents, and then stirring the oil phase and the aqueous phase with a stirrer (stirring speed: about 500 rpm/stirring period: about 1hour). All of these steps were performed at room temperature. After the stirring, a liquid composition was yielded in which a polymeric reversed micelle was dispersed.

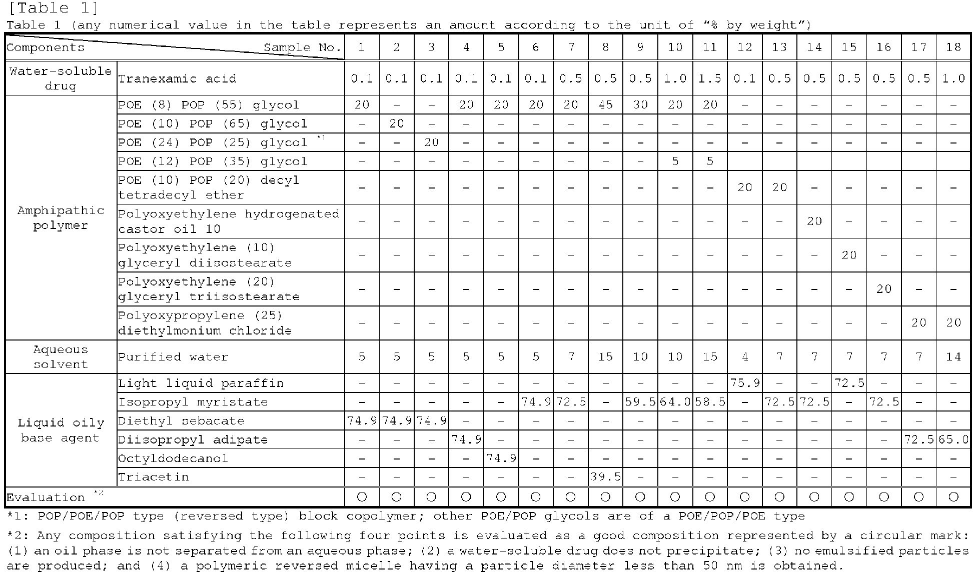

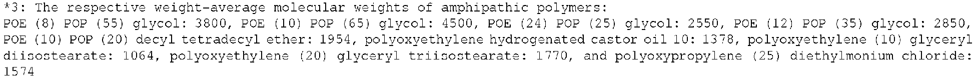

The compositions in Table 1 each satisfied the following four points: [1] an oil phase is not separated from an aqueous phase; [2] a water-soluble drug does not precipitate; [3] only a polymeric reversed micelle is produced (no emulsified particles are produced); and [4] a polymeric reversed micelle having a particle diameter less than 50 nm is obtained. The particle diameter was measured at room temperature by the dynamic light scattering method, using, as measuring instruments, a particle diameter measuring system (ELSZ series) manufactured by Otsuka Electronics Co., Ltd., and an instrument, Nicomp 380ZLS-S, manufactured by Particle Sizing Systems Japan Co., Ltd.
About the samples 1 to 18, compositions each containing a reversed micelle having an average particle diameter at a level of 1 nm to 40 nm were able to be prepared (for example, the average particle diameter of the reversed micelle was 40.7 nm about the sample 7, 33.8 nm about the sample 6, 25.4 nm about the sample 3, 24.8 nm about the sample 14, 16.8 nm about the sample 12, 13.9 nm about the sample 18, 12.5 nm about the sample 13, 10.9 nm about the sample 15, and 1.3 nm about the sample 17. About the sample 16, a composition was obtained in which its reversed micelle had two-peak particle diameters of 5.3 nm and 11.7 nm.)
The polymeric reversed micelle compositions of the samples 1 to 18 were each transparent.
Furthermore, the producing process was changed to attempt to produce polymeric reversed micelle compositions. Specifically, compositions based on the samples 7 and 8 were each produced by stirring its oily base agent and an aqueous phase prepared by adding, to its aqueous solvent, its water-soluble drug and its amphipathic polymer and then blending these with each other. In this case also, a polymeric reversed micelle composition satisfying the above-mentioned four points was able to be obtained.

As shown by the compositions of the samples 6 and 7, in the case of using 20% by weight of polyoxyethylene (8) polyoxypropylene (55) glycol as their amphipathic polymer and using isopropyl myristate as their oily base agent, and further increasing the proportion of their water-soluble drug (tranexamic acid) from 0.1 to 0.5% by weight, the water-soluble drug precipitated favorably less easily when the proportion of the aqueous solvent (purified water) was increased from 5% to 7% by weight.
As shown by the compositions of the samples 7 and 9, in the case of setting the proportion of their water-soluble drug to 0.5% by weight, and further increasing the proportion of their aqueous solvent from 7 to 10% by weight, emulsified particles were produced favorably with less easily when the proportion of their amphipathic polymer was increased from 20% to 30% by weight.
As shown by the compositions of the samples 10 and 11, in the case of setting the proportion of their water-soluble drug to a larger value (1.0 or 1.5% by weight), the water-soluble drug precipitated favorably less easily when the following were together used as their amphipathic polymers and the proportion of their aqueous solvent was increased to 10% by weight or 15% by weight: polyoxyethylene (8) polyoxypropylene (55) glycol; and polyoxyethylene (12) polyoxypropylene (35) glycol (20% by weight + 5% by weight).

### [Example 2] Skin permeability test:

A skin permeability test was made about the sample 7 produced in Example 1 and Comparative Examples 1 to 4 shown in Table 2.

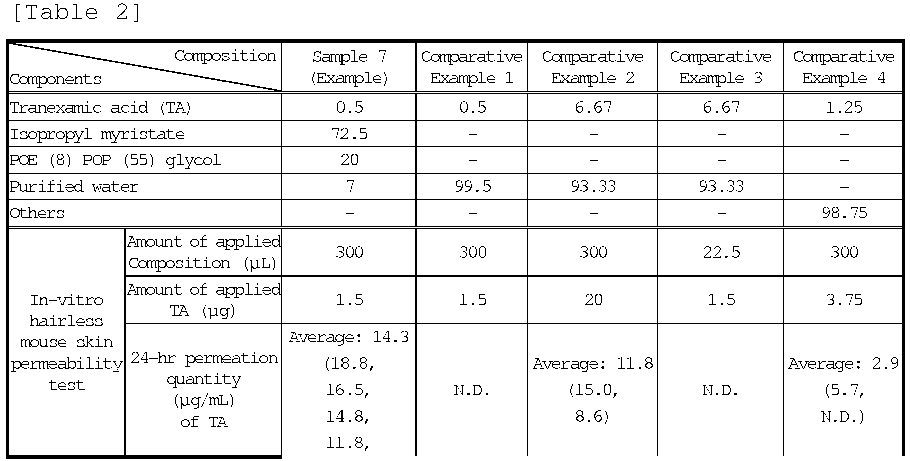

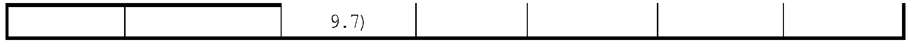

As shown in Table 2, Comparative Example 1 was an aqueous solution containing 0.5% by weight of tranexamic acid.
Comparative Example 2 was an aqueous solution in which the thermodynamic activity of tranexamic acid was consistent with that of the sample 7 (tranexamic acid : water = 1 : 14). It is generally considered that as the concentration of a drug in a solvent is closer to the saturation concentration thereof, the drug is higher in thermodynamic activity to be more advantageous for percutaneous absorption. Since tranexamic acid (TA) is a water-soluble drug, the polymeric reversed micelle solution wherein TA : water = 1 : 14 (sample 7), and the aqueous solution wherein TA : water = 1 : 200 (Comparative Example 1) were largely different from each other in thermodynamic activity even when the respective TA concentrations in the compositions were equal to each other, 0.5% by weight. Thus, in Comparative Example 2, the ratio of tranexamic acid to water was set to 1 : 14, thus making the thermodynamic activity of tranexamic acid consistent with that of the sample 7.
Comparative Example 3 was a sample equivalent to Comparative Example 2, and the amount of tranexamic acid in the applied sample was made consistent with that of the sample 7.
The compositions of Comparative Examples 1 to 3 were prepared by adding tranexamic acid to purified water and stirring the resultant with a stirrer to dissolve the acid therein.
Comparative Example 4 was a commercially available product containing tranexamic acid. Components other than tranexamic acid were potassium 4-methoxysalicylate, acetic acid DL-α-tocopherol, and dipotassium glycyrrhizinate (these were effective components); and 1-menthol, 3-O-ethyl ascorbic acid, purified water, concentrated glycerin, dipropylene glycol, and others.

The sample 7 and Comparative Examples 1 to 4 were used to make an in-vitro hairless mouse skin permeability test. The test was made by filling receptor-side parts of a Franz diffusion cell (opening area: 0.64 cm²) with a receptor liquid (PBS buffer, volume: 5 mL, and liquid temperature: 32°C), setting a hairless mouse normal skin thereto, setting donor-side parts thereto, and applying each of the compositions thereon. After 24 hours, the receptor liquid was analyzed by high-performance liquid chromatography to calculate the 24-hour skin permeability quantity (µg/mL). About the sample 7, the test was made 5 times. About each of Comparative Examples 1 to 4, the test was made 2 times. The average value thereof was calculated. In the table, "N. D." shows that the permeation quantity of tranexamic acid was a detection limit thereof, or less.

As shown in Table 2, about the permeation quantity of tranexamic acid (TA), the composition of the sample 7 according to the present invention showed a high value of 14.3 µg/mL on average. In contrast thereto, the TA permeation quantity of the aqueous TA solution (Comparative Example 1) having the same TA concentration was the detection limit or less. In the case of using the aqueous solution having a TA concentration of 6.67% by weight (13.3 times the concentration in the sample 7), Comparative Example 2, which was equal in the applied amount of the composition to the sample 7 (in other words, the applied amount of TA itself was 13.3 times that of the sample 7), was also lower in TA permeation quantity than the sample 7. In Comparative Example 3, in which the applied amount of TA itself was equal to that of the sample 7, the TA permeation quantity was the detection limit or less. Furthermore, when compared with the commercially available product (Comparative Example 4), which contained TA in a higher concentration than the sample 7, the composition of the sample 7 according to the present invention showed a TA permeation quantity about 5 times that of Comparative Example 4.
From this matter, it has been actually verified that the polymeric-reversed-micelle-containing composition according to the present invention makes it possible to deliver a water-soluble drug in its reversed micelle into the skin with a highly efficiency. It is therefore expected that even when the composition according to the present invention is low in the concentration of a drug in this composition, the composition produces a high pharmacological effect.

### [Example 3] Consideration about encapsulating-in percentage of water-soluble drug:

About the polymeric reversed micelle compositions produced in the above-mentioned examples, their outside phase was an oil phase and their inside phase was an aqueous phase. Thus, if their water-soluble drug was present in the outside phase (oil phase) for a reason that the water-soluble drug was not encapsulated in the reversed micelle, a reason that the water-soluble drug encapsulated in the reversed micelle leaked outside from the reversed micelle and/or other reasons, the precipitation of the drug would have been able to be verified with the naked eye or through microscopic observation. By contrast, if the polymeric reversed micelle compositions showed a transparent external appearance, their water-soluble drug should have been encapsulated in their reversed micelle with a probability of 100% (or approximately 100%). The polymeric reversed micelle compositions (the samples 1 to 18) produced in Example 1 were each transparent, and no precipitation was recognized even through microscopic observation. It can be therefore presumed that the reversed micelle in each of these compositions encapsulated therein the water-soluble drug in a encapsulating-in percentage of 100%.
In particular, in the samples 7 to 11, and 13 to 18, their water-soluble drug was able to be completely encapsulated in their reversed micelle although the water-soluble drug was relatively high in concentration.
It is therefore considered that the polymeric reversed micelle related to the present invention has a far higher drug-encapsulating-in percentage than liposome. For example, the drug-encapsulating-in percentage of a liposome disclosed in JP-A-6-2329533 is 80% or less, and that of a liposome disclosed in JP-A-6-2247842 is 61% or less. That of a liposome disclosed in JP-A-2010-248171 is 71% or less. That of a liposome disclosed in JP-A-2010-222282 is 61% or less. In short, a drug-encapsulating-in percentage close to 100% cannot be attained.

The particle diameter of the polymeric reversed micelle contained in, for example, the sample 7 used in Example 2 was 40.7 nm. However, it is very difficult to produce a liposome having such a particle diameter. Even when such a liposome can be produced, the liposome becomes very low in drug-encapsulating-in percentage. Accordingly, the present invention makes it possible to produce a polymeric reversed micelle having a smaller particle diameter than that of any other carrier, such as liposome, the diameter being advantageous for percutaneous absorption, and further attain a higher drug-encapsulating-in percentage.
The polymeric reversed micelle related to the present invention is characterized by being less easily broken than lower-molecular-weight micelles when diluted in an administration route thereof.
Accordingly, the polymeric reversed micelle related to the present invention can make the percutaneous absorptiveness of a water-soluble drug therein remarkably higher than drug-delivering carriers that have been hitherto used for the skin.

### INDUSTRIAL APPLICABILITY

The producing method according to the present invention makes it possible to produce, through a simple step, a dermal composition containing a polymeric reversed micelle encapsulating therein a water-soluble drug. This polymeric reversed micelle has a high encapsulating-in percentage of a water-soluble drug. Even when the micelles are diluted in an administration route thereof, the micelles are not easily broken. Thus, the composition is very suitable for delivering a water-soluble drug into the skin.

## Claims

1. A method for producing a dermal composition comprising a polymeric reversed micelle in which a water-soluble drug is encapsulated, comprising:
the step of blending an oil phase comprising an amphipathic polymer, which is having a hydrophilic segment and a hydrophobic segment, in an oily base agent; with an aqueous phase comprising a water-soluble drug in an aqueous solvent, or
blending an oily base agent with an aqueous phase comprising the above-defined amphipathic polymer and a water-soluble drug in an aqueous solvent,
thereby producing the polymeric reversed micelle in which the hydrophilic segment is a core, the hydrophobic segment is a shell and the water-soluble drug is encapsulated.

2. The method according to claim 1, wherein the amphipathic polymer is one or more selected from the group consisting of polyoxyethylene polyoxypropylene glycols, polyoxyethylene polyoxypropylene alkyl ethers, aliphatic acid esters of any polyoxyethylene glyceryl ether, polyoxyethylene hydrogenated castor oils, and quaternary ammonium salts of any polyoxypropylene.

3. The method according to claim 1 or 2, wherein the amphipathic polymer is one or more selected from the group consisting of polyoxyethylene (8) polyoxypropylene (55) glycol, polyoxyethylene (30) polyoxypropylene (35) glycol, polyoxyethylene (24) polyoxypropylene (25) glycol, polyoxyethylene (12) polyoxypropylene (35) glycol, polyoxyethylene (10) polyoxypropylene (65) glycol, polyoxyethylene (3) polyoxypropylene (17) glycol, and polyoxyethylene (5) polyoxypropylene (30) glycol; polyoxyethylene (17) polyoxypropylene (17) butyl ether, polyoxyethylene (30) polyoxypropylene (30) butyl ether, polyoxyethylene (37) polyoxypropylene (38) butyl ether, polyoxyethylene (25) polyoxypropylene (25) lauryl ether, polyoxyethylene (3) polyoxypropylene (34) stearyl ether, and polyoxyethylene (10) polyoxypropylene (20) decyl tetradecyl ether;
polyoxyethylene (10) glyceryl diisostearate, and polyoxyethylene (20) glyceryl triisostearate;
polyoxyethylene hydrogenated castor oil 10; and polyoxypropylene (25) diethylmonium chloride.

4. The method according to any one of claims 1 to 3, wherein the oily base agent is one or more selected from the group consisting of hydrocarbons, aliphatic acid esters, higher alcohols, and highly polar oils.

5. The method according to any one of claims 1 to 4, wherein the oily base agent is one or more selected from the group consisting of liquid paraffin, isopropyl myristate, diethyl sebacate, diisopropyl adipate, octyldodecanol, triacetin, and ethylene glycol salicylate.

6. The method according to any one of claims 1 to 5, wherein in the manner of setting the contents of the amphipathic polymer, the oily base agent, the aqueous solvent and the water-soluble drug in the dermal composition into the range of 15 to 50% by weight, that of 35 to 80% by weight, that of 3 to 20% by weight, and that of 0.05 to 3% by weight, respectively, the components are blended with each other.

7. The method according to any one of claims 1 to 6, wherein the amphipathic polymer is one or more selected from the group consisting of polyoxyethylene (8) polyoxypropylene (55) glycol, polyoxyethylene (10) polyoxypropylene (65) glycol, polyoxyethylene (24) polyoxypropylene (25) glycol, polyoxyethylene (12) polyoxypropylene (35) glycol, polyoxyethylene (10) polyoxypropylene (20) decyl tetradecyl ether, polyoxyethylene (10) glyceryl diisostearate, polyoxyethylene (20) glyceryl triisostearate, polyoxyethylene hydrogenated castor oil 10, and polyoxypropylene (25) diethylmonium chloride, and
the oily base agent is one or more selected from the group consisting of liquid paraffin, isopropyl myristate, diethyl sebacate, diisopropyl adipate, octyldodecanol, triacetin, and ethylene glycol salicylate.

8. The method according to any one of claims 1 to 7, wherein the aqueous solvent consists of water and/or a polyhydric alcohol.

9. The method according to any one of claims 1 to 8, wherein the polymeric reversed micelle has a particle diameter less than 50 nm.

10. The method according to any one of claims 1, and 4 to 9, wherein the amphipathic polymer is a polyoxyethylene polyoxypropylene glycol.

11. The method according to claim 10, wherein the polyoxyethylene polyoxypropylene glycol is one or more selected from the group consisting of polyoxyethylene (8) polyoxypropylene (55) glycol, polyoxyethylene (30) polyoxypropylene (35) glycol, polyoxyethylene (24) polyoxypropylene (25) glycol, polyoxyethylene (12) polyoxypropylene (35) glycol, polyoxyethylene (10) polyoxypropylene (65) glycol, polyoxyethylene (3) polyoxypropylene (17) glycol, and polyoxyethylene (5) polyoxypropylene (30) glycol.

12. The method according to any one of claims 1, and 4 to 9, wherein the amphipathic polymer is a polyoxyethylene polyoxypropylene alkyl ether.

13. The method according to claim 12, wherein the polyoxyethylene polyoxypropylene alkyl ether is one or more selected from the group consisting of polyoxyethylene (17) polyoxypropylene (17) butyl ether, polyoxyethylene (30) polyoxypropylene (30) butyl ether, polyoxyethylene (37) polyoxypropylene (38) butyl ether, polyoxyethylene (25) polyoxypropylene (25) lauryl ether, polyoxyethylene (3) polyoxypropylene (34) stearyl ether, and polyoxyethylene (10) polyoxypropylene (20) decyl tetradecyl ether.

14. The method according to any one of claims 1, and 4 to 9, wherein the amphipathic polymer is one or more selected from the group consisting of aliphatic acid esters of any polyoxyethylene glyceryl ether, polyoxyethylene hydrogenated castor oils, and quaternary ammonium salts of any polyoxypropylene.

15. The method according to claim 14, wherein the amphipathic polymer is one or more selected from the group consisting of polyoxyethylene (10) glyceryl diisostearate, polyoxyethylene (20) glyceryl triisostearate, polyoxyethylene hydrogenated castor oil 10, and polyoxypropylene (25) diethylmonium chloride.

16. A dermal composition, comprising a polymeric reversed micelle comprising an amphipathic polymer having a hydrophilic segment and a hydrophobic segment,
wherein the polymeric reversed micelle is a micelle in which the hydrophilic segment is a core, the hydrophobic segment is a shell and a water-soluble drug is encapsulated.

17. The composition according to claim 16, wherein the amphipathic polymer is one or more selected from the group consisting of polyoxyethylene polyoxypropylene glycols, polyoxyethylene polyoxypropylene alkyl ethers, aliphatic acid esters of any polyoxyethylene glyceryl ether, polyoxyethylene hydrogenated castor oils, and quaternary ammonium salts of any polyoxypropylene.

18. The composition according to 16 or 17, wherein the amphipathic polymer is one or more selected from the group consisting of polyoxyethylene (8) polyoxypropylene (55) glycol, polyoxyethylene (30) polyoxypropylene (35) glycol, polyoxyethylene (24) polyoxypropylene (25) glycol, polyoxyethylene (12) polyoxypropylene (35) glycol, polyoxyethylene (10) polyoxypropylene (65) glycol, polyoxyethylene (3) polyoxypropylene (17) glycol, polyoxyethylene (5) polyoxypropylene (30) glycol, polyoxyethylene (17) polyoxypropylene (17) butyl ether, polyoxyethylene (30) polyoxypropylene (30) butyl ether, polyoxyethylene (37) polyoxypropylene (38) butyl ether, polyoxyethylene (25) polyoxypropylene (25) lauryl ether, polyoxyethylene (3) polyoxypropylene (34) stearyl ether, and polyoxyethylene (10) polyoxypropylene (20) decyl tetradecyl ether;
polyoxyethylene (10) glyceryl diisostearate, and polyoxyethylene (20) glyceryl triisostearate;
polyoxyethylene hydrogenated castor oil 10; and
polyoxypropylene (25) diethylmonium chloride.

19. The composition according to any one of claims 16 to 18, comprising one or more oily base agents selected from the group consisting of hydrocarbons, aliphatic acid esters, higher alcohols, and highly polar oils.

20. The composition according to claim 19, wherein the oily base agent(s) is/are one or more selected from the group consisting of liquid paraffin, isopropyl myristate, diethyl sebacate, diisopropyl adipate, octyldodecanol, triacetin, and ethylene glycol salicylate.
